# EUROPEAN PATENT SPECIFICATION

(11) **EP 0 696 212 B1**
(45) Date of publication and mention of the grant of the patent: **07.11.2001**
(21) Application number: 94903414.4
(22) Date of filing: 03.12.1993
(51) Int. Cl.: A61M 5/178, A61M 5/32, A61M 25/06

(54) **INTRAVENOUS CANNULA ASSEMBLY**
INTRAVENöSE KANüLENEINHEIT
CANULE INTRAVEINEUSE

(30) Priority: 04.12.1992 IL 10397392
(43) Date of publication of application: 14.02.1996
(73) Proprietor: TRAVENOL LABORATORIES (ISRAEL) LTD., Ashdod 77100 (IL)
(72) Inventor: KRAUS, Menahem, 76 100 Rehovot (IL); SHEMESH, Eli, 77 100 Ashdod (IL); ROGEL, Eitan, 31 072 Haifa (IL)
(74) Representative: Modiano, Guido, Dr.-Ing.
(86) International application number: US9311719
(87) International publication number: WO9413341

(56) References cited:
- WO-A-93/08865
- US-A- 4 762 516
- US-A- 4 832 696
- US-A- 4 834 718
- US-A- 4 934 718
- US-A- 4 944 728
- US-A- 4 986 819
- US-A- 5 019 049

## Description

### TECHNICAL FIELD

The present invention relates to an intravenous cannula assembly, and particularly to an assembly which includes a cannula and a guide needle which is used for piercing the subject's skin and for guiding the cannula through the so-formed opening in the skin, after which the guide needle is withdrawn. The cannula so inserted into the subject's skin is then used for draining fluid or for introducing a medication. The invention is particularly directed to an intravenous cannula assembly which provides protection for healthcare personnel from inadvertent contact with the end of the guide needle after it has been contaminated by the subject's blood.

### BACKGROUND ART

According to current practise, venous access for draining fluid or for introducing a medication is generally established as follows: A plastic cannula, e.g., of Teflon (Reg.TM) or polyurethane, containing a hollow, metal guide-needle, is inserted into the subject's vein. Once blood flow through the needle is observed, the needle is withdrawn while holding the cannula inside the vein. The needle is then discarded while preventing blood outflow from the cannula, and a connection is made from the cannula to the intravenous set.

Once the neeedle has pierced the subject's skin, it becomes contaminated with the subject's blood, and therefore there is a danger of infecting healthcare personnel subsequently coming in contact with the needle. This danger is particularly real because of the many simultaneous operations involved in intravenous infusion and in emergency situations which may arise and which may prevent careful attention to the safety of the healthcare personnel. This danger can also be health-threatening because of the possibility of contracting AIDS in this manner.

Many types of protective devices have been developed to reduce the danger, but none of the devices which are now commercially available is entirely satisfactory. For example, some of the cannula assemblies developed for this purpose hamper or substantially change the common practise of establishing venous access for intravenous infusion. Others do not provide automatic protection, but rather necessitate a special needle protection step which may often be sidestepped. Still others require cannula assemblies which are substantially larger than the products used in the current practise.

For example, one commercially-available product includes a cover which covers the needle before it is inserted and which is subsequently removed and then used to protect the extracted needle. However, this product substantially changes the common procedure for establishing venous access, and moreover it is not automatic but rather requires the operator to take a special protection step, namely to reapply the cover to the extracted needle. In addition, the product is long and cumbersome.

US Patent 4,955,866 describes a protective cover tethered to a needle, the cover being applied to the needle tip after it has pierced the skin. However, this device also requires special attention of the user; moreover, it protects only the needle tip from contact and not the remainder of the needle which may also be contaminated.

Another product, described in US Patent 4,832,696, includes a cylinder which receives the needle after its removal. However, this device is substantially larger than the current products, its length by necessity being more than twice the length of the needle, which makes it large and cumbersome to use.

US Patent 4,897,083 describes another device, in the form of a syringe, with a needle protected by a telescoping mechanism. However, in this device the mechanism must be manually contracted before use, and manually extended after use. Such a non-automatic operation is highly inefficient and undesirable.

Known from WO-A-9308865, which is a document cited under Art. 54(3) EPC, is a needle catheter for endovasal use, including a metallic needle-guide, with an end grip and a catheter with related grip or end casing covering the needle and having an extensible element situated between the grip of the needle and the casing of the catheter, in such a way as to cover the needle when this is retracted; also by the fact of having a blocking system for the needle in the retracted position, as well as the means of intercepting the flow in the lumen of the casing of the catheter.

From USP 4,944,728 is known an intravenous catheter placement device which includes a tubular catheter section concentrically surrounding an elongated tubular needle which has a bias cut point projecting from one end of the catheter section with the bias cut point of the needle being used to lead the catheter section into a blood vessel. A protective sheath subassembly is connected to the needle and to the catheter section to permit the needle to be withdrawn through the tubular catheter section into a protective rigid tubular sheath, leaving the catheter section emplaced in the blood vessel. Locking elements on the protective tubular sheath subassembly lock the needle in the protected position.

US -A-4,834,718 discloses a clinical needle apparatus comprising: a needle having a pointed forward end adapted to intentionally puncture the skin of a patient; handle means secured to the needle rearwardly of the pointed end and adapted for manually pulling said needle rearwardly with a motion having only a rearward translational component in order to withdraw said needle from an intentional puncture site; protective housing means having an interior needle-receiving passage therein; said needle being received within said passage with said pointed end extending forwardly out of said passage, and being mounted therein in manner to be moved rearwardly relative to said protective housing means; said passage being long enough to permit said pointed end to be withdrawn rearwardly into said passage to prevent accidental punctures from occurring after said intentional puncture; and latch means on said handle means and said protective housing means interengaging upon rearward motion of said handle means and said needle relative to said protective housing to prevent subsequent accidental forward motion of said handle means and said needle relative to said protective housing means; said latch means being positioned so as to interengage only after said pointed end of said needle has entered said passage.

An object of the present invention is to provide an intravenous cannula assembly having advantages in the above respects.

### DISCLOSURE OF INVENTION

According to the present invention, there is provided an intravenous cannula assembly, comprising: a cannula having a proximal end insertable through an opening in a subject's skin for draining fluid or for introducing a medication via its distal end; a guide needle movable to an operative position with respect to the cannula and having a proximal end for piercing the subject's skin and for guiding the proximal end of the cannula through the subject's skin, the guide needle also being movable to a retracted position via the distal end of the cannula for removal therefrom; and a protective enclosure for enclosing the guide needle when moved to its retracted position; the protective enclosure comprising a plurality of sections having a total length to completely enclose the guide needle when in its retracted position; the plurality of sections being movable from a compact nested condition in the operative position of the guide needle, to an extended protective condition in the retracted position of the guide needle; the sections including an outer section to which the distal end of the needle is secured, and an inner section detachably secured to a cannula hub adjacent the distal end of the cannula; wherein the distal end of the cannula is secured to one end of the cannula hub, the opposite end of the hub having a rotatable connection with the inner section of the protective enclosure, the inner section being rotatable with respect to the cannula hub to attach the inner section to the cannula hub, and to detach the inner section from the cannula hub;
rotatable coupling means which couples together the sections for rotation only in the extended condition of the enclosure, said rotatable coupling means including a tooth at the proximal end of an intermediate section co-operable with an annular array of teeth at the distal end of said inner section; and
a pair of diametrically opposed ribs fixed to the inner section of the protective enclosure and a pair of diametrically opposed ribs fixed to the cannula hub, one of said pair of ribs being formed with radially extending projections receivable within recesses formed in said other pair of ribs, such that the protective enclosure, with the guide needle enclosed therein, may be rotated in either direction to detach it from said cannula hub.

Three embodiments are described below for purposes of example. In all three described embodiments, the protective enclosure includes blocking means carried by the plurality of sections of the protective enclosure permitting movement of the sections from the compact nested condition to the extended protective condition, but blocking movement of the sections from the extended protective condition to the compact nested condition. Also, in all three described embodiments, the protective enclosure is a three-section construction, including an intermediate section between its inner and outer sections.

As will be described more particularly below, an intravenous cannula assembly constructed in accordance with the foregoing features provides a number of important advantages over the known devices. Thus, it can be constructed so as not to be substantially larger than current products. Moreover, it provides automatic protection when withdrawing the needle from the cannula and does not require a special needle protection step, as distinguished for example from the device described in US Patent 4,897,083 which requires manual contraction of the telescoping mechanism before use, and manual extension after use in order to provide the protection against contact with the needle. Further, the device prevents re-extraction of the needle from its contracted protected condition. In addition, it is simple to use and inexpensive to manufacture.

Further features and advantages of the invention will be apparent from the description below.

### BRIEF DESCRIPTION OF DRAWINGS

The invention is herein described, by way of example only, with reference to the accompanying drawings and particularly to drawings 12-23, wherein:
Fig. 1 is a longitudinal sectional view illustrating one form of intravenous cannula assembly, the assembly being shown with the guide needle in its operative position preparatory for use of the assembly;
Fig. 2 is a view similar to that of Fig. 1 but showing the needle in its retracted position after use of the assembly;
Fig. 3 is an enlarged fragmentary view of a portion of the assembly of Fig. 2;
Figs. 4 and 5 are views, corresponding to those of Figs. 1 and 2, respectively, illustrating a second form of intravenous cannula assembly;
Fig. 6 is a sectional view of Fig. 5 along line VI--VI;
Fig. 7 is a view similar to that of Fig. 5 but showing how the three sections of the protective enclosure are assembled;
Figs. 8 and 8a-8c are enlarged views more particularly illustrating the construction of the inner section of the protective enclosure in Figs. 4-7;
Figs. 9, 9a and 9b are enlarged views more particularly illustrating the construction of the intermediate section of the protective enclosure in Figs. 4-7;
Figs. 10, 10a and 10b are enlarged views more particularly illustrating the construction of the outer section of the protective enclosure in Figs. 4-7;
Figs. 11, 11a and 11b more particularly illustrate the Leuer connection in the assembly of Figs. 4-10b;
Figs. 12 and 13 are sectional views illustrating a third form of cannula assembly constructed in accordance with the invention and illustrating the guide needle in its operative position, Fig. 13 being a sectional view along line XIII-XIII of Fig. 12;
Figs. 14 and 15 are corresponding views of the cannula assembly of Figs. 12 and 13, but showing the assembly in the extended protective condition of the enclosure;
Fig. 16 is a sectional view of Fig. 14;
Fig. 17 illustrates the inner section of the enclosure of Figs. 12-15; Fig. 18 is an end view thereof;
Fig. 19 illustrates the intermediate section of the enclosure of Figs. 12-15; Fig. 20 is an end view thereof;
Fig. 21 is a sectional view illustrating the outer part of the enclosure of Figs. 12-15; Fig. 22 is a transverse section thereof;
and Fig. 23 is an end view illustrating the Leuer coupling used in the cannula assembly of Figs. 12-15.

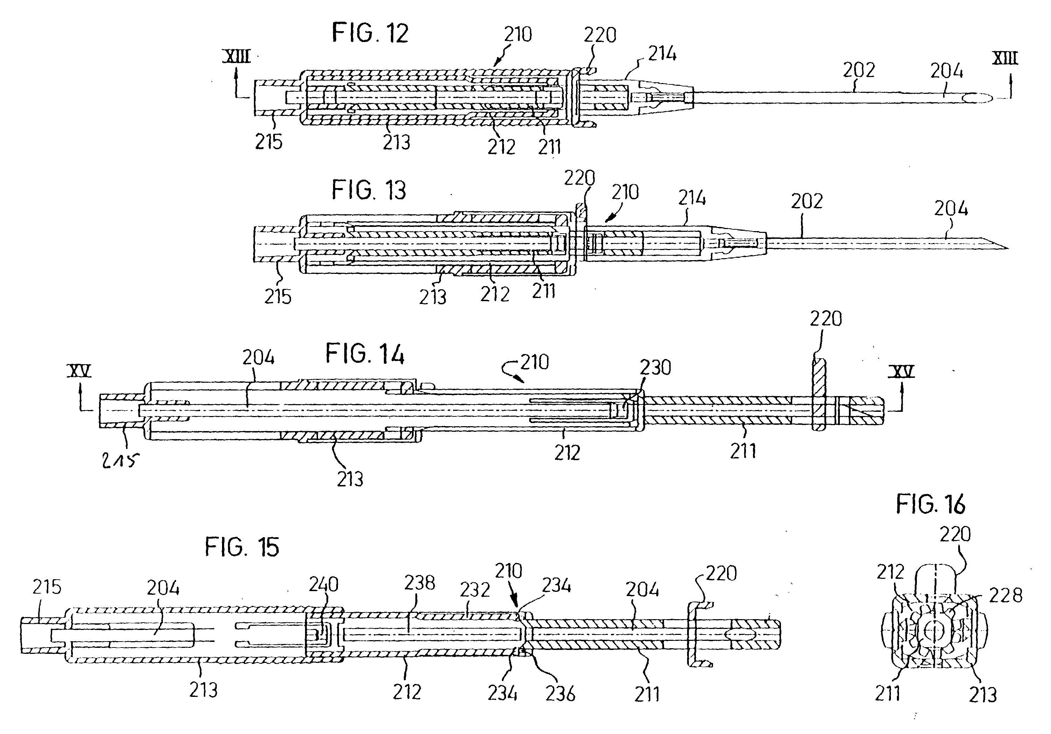

### MODE FOR CARRYING OUT THE INVENTION (SEE FIGURES 12-23)

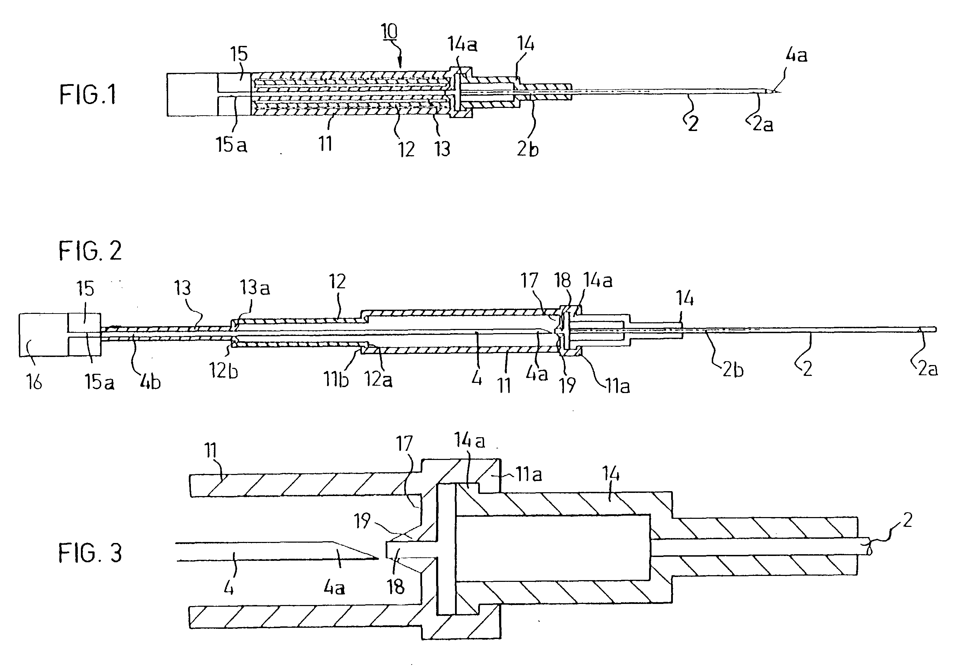

The intravenous cannula assembly illustrated in Figs. 1-3 of the drawings is of the type which includes an outer plastic cannula 2 and an inner, hollow, metal guide-needle 4 movable within the cannula 2. In the operative position of the guide needle 4 illustrated in Fig. 1, its sharpened proximal end 4a is used for piercing the subject's skin and for guiding the proximal end 2a of the cannula into the opening so formed in the subject's skin. Once blood flow through the needle 4 is observed, the needle is moved to its retracted position through the distal end 2b of the cannula 2, as shown in Fig. 2. The needle is then detached from the cannula and discarded, leaving the cannula 2 inside the vein. The cannula is then connected to the intravenous set for draining a fluid (e.g., blood) from the subject's body, or for introducing a medication into the subject's body.

Before the needle 4 is used for piercing the subject's skin, it is in a sterilized condition, and therefore there is no danger of contaminating healthcare pesonnel. However, once the needle has pierced the subject's skin, it becomes contaminated with the patient's blood, and therefore it must be protected from inadvertent needle sticks or other contact with healthcare personnel.

For the latter purpose, the assembly illustrated in the drawings includes a protective enclosure, generally designated 10, for enclosing the guide needle 4 when moved to its retracted position (Fig. 2). The protective enclosure 10 includes a plurality of sections, 11, 12 and 13. These sections are all of rectangular cross-section, decreasing in transverse dimensions from the inner section 11 to the outer section 13. That is, the transverse dimensions of the intermediate section 12 are smaller than those of the inner section 11, but larger than those of the outer section 13.

In the normal operative position of the guide needle 4 as illustrated in Fig. 1, sections 11-13 are in a contracted, nesting, telescoping relation to each other; however, in the retracted position of the needle, as illustrated in Fig. 2, these sections are extended to a total length to completely enclose the extracted needle, and thereby to prevent contact with it by healthcare personnel subsequently handling the extracted needle.

More particularly, the distal end 2b of the cannula 2 is secured to one end of a cannula hub 14. The opposite end of the cannula hub is formed with an outturned annular rib 14a which detachably engages an inturned annular rib 11a of the inner telescoping section 11. The outer end of telescoping section 11 is similarly formed with an inturned annular rib 11b engageable with an outturned annular rib 12a formed in the inner end of the intermediate telescoping section 12. The outer end of telescoping section 12 is similarly formed with an inturned annular rib 12b engageable with an outturned rib 13a formed in the inner end of the outer telescoping section 13.

The distal end 4b of the hollow needle 4 is secured to a needle hub 15, which in turn is secured to the outer telescoping section 13 of the protective enclosure 10. The needle hub 15 is formed with a bore 15a aligned with the hollow interior of needle 4. This bore is closed by a plug 16, which is porous and hydrophobic enabling the venting of air and preventing blood from seeping out.

The inner end of the inner telescoping section 11 is closed by an end wall 17 (fig.3) formed with a center bore 18 having a diameter substantially equal to the outer diameter of the hollow needle 4. The outer surface of end wall 17 includes a conical protrusion 19 through which bore 18 is formed. Protrusion 19 decreases in diameter in the outer direction, i.e., in the direction of the intermediate and outer telescoping sections 12 and 13. This arrangement serves as a safety means permitting the guide needle 4 to move through bore 18 in one direction, from the operative position of the needle (Fig. 1) to its retracted position (Fig. 2), but not in the opposite direction, i.e., from its retracted position to its operative position.

The cannula assembly illustrated in Figs. 1-3 of the drawings is used in the following manner:

The assembly is normally supplied in the condition illustrated in Fig. 1. In this normal condition of the assembly, the guide needle 4 is in its operative position with its proximal end 4a projecting through the proximal end 2a of the cannula 2, and the protective enclosure 10 is in its contracted condition, i.e., telescoping sections 12 and 13 both telescoped within section 11. The assembly would normally be packaged and sterilized in this condition as illustrated in Fig. 1.

When the assembly is to be used for drawing fluid (e.g., blood), or for administering a medication to a subject, the proximal end 4a of the needle 4, together with the proximal end 2a of the cannula 2, is inserted into the subject's vein. Successful insertion of the needle and cannula is noted by the flow of blood through the needle and the bore 15a in the needle hub 15. As soon as blood flow is observed, the outer telescoping section 13, together with the needle hub 15, is moved outwardly (leftwardly, Fig. 1) to extract the needle from the subject and also from the cannula 2. The outer telescoping section 13 is moved outwardly until its annular rib 13a engages annular rib 12b of the intermediate telescoping section 12, thereby causing that section to move outwardly until its annular rib 12a engages annular rib 11b of the innermost telescoping section 11.

During this expansion of the three telescoping sections 11, 12 and 13 of the protective enclosure 10, needle 4, being secured to the outermost telescoping section 13, is moved through the tight-fitting bore 18 out from the cannula 2 and its hub 14, so that in the fully extended condition of the protective enclosure, the needle 4 is completely enclosed within the three telescoping sections 11-13, as shown in Fig. 2. The tight-fitting bore 18 formed in the end wall 17 of the innermost telescoping section 11, and the conical protrusion 19 through which bore 18 is formed, prevent the needle 4 from being moved in the reverse direction, i.e., back into the cannula 2.

When the needle 4 has thus been moved to its fully retracted position as illustrated in Fig. 2, wherein it is completely enclosed within the three telescoping sections 11-13 of the protective enclosure 10, the protective enclosure 10 is easily detached from the cannula hub 14 and discarded. The cannula hub may then be connected to a device for receiving fluid (e.g., blood) drained from the subject, or to a device for supplying fluid (e.g., medication) to be introduced into the subject via the cannula 2.

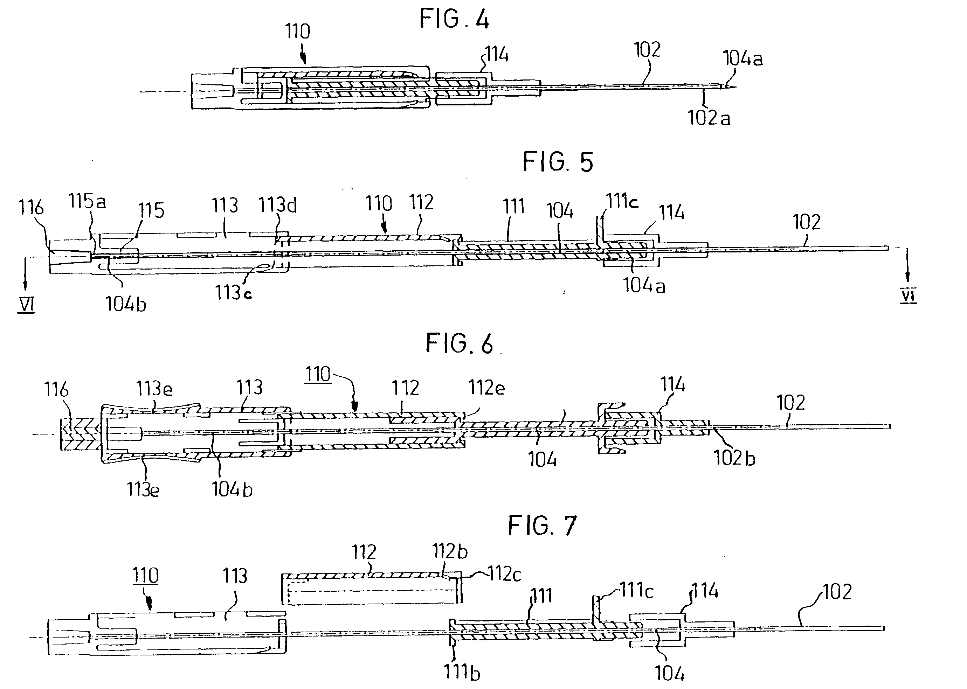

The intravenous cannula assembly illustrated in Figs. 4-11b is of a similar type as that of Figs. 1-3. It includes an outer plastic cannula 102, an inner hollow metal guide needle 104, and a protective enclosure 110 for enclosing the guide needle when moved to its retracted position, as described above with respect to Figs. 1-3. The protective enclosure 110 also includes a plurality of telescoping sections 111, 112 and 113, also of rectangular cross-section, but in this case the transverse dimensions of the sections increase from the inner section 111 to the outer section 113; that is, the transverse dimensions of the intermediate section 112 are larger than those of the inner section 111 and smaller than those of the outer section 113, which is opposite to the arrangement in Figs. 1-3.

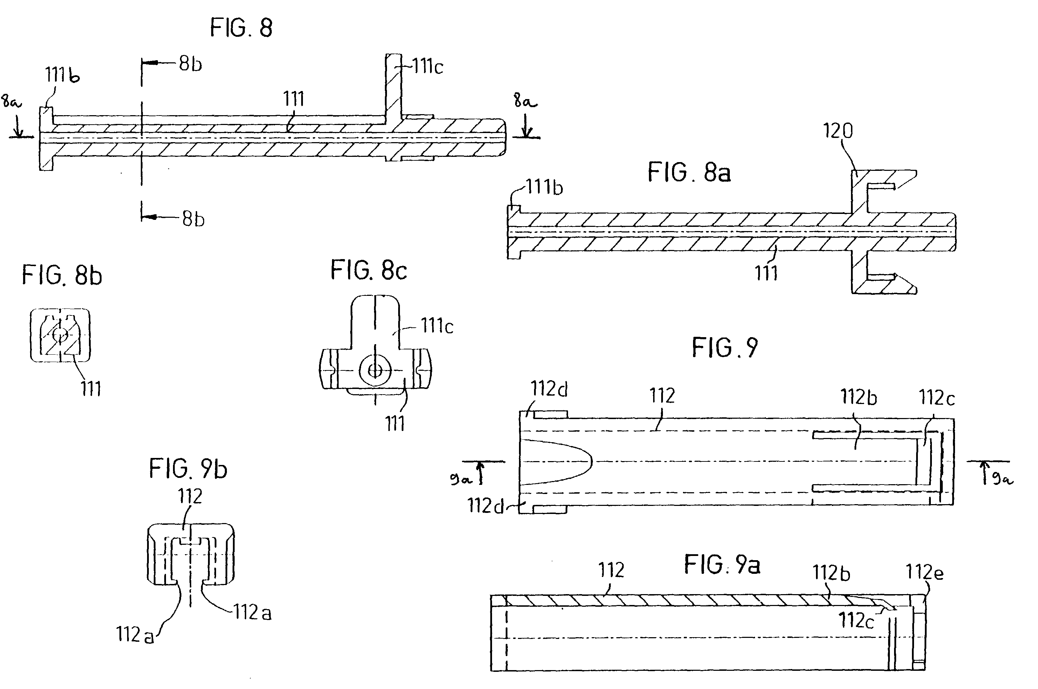

The intermediate section 112 is open at one side, as shown particularly in Figs. 9a and 9b, and is formed with a pair of inwardly-directed ribs 112a adapted to be snapped over the outer face of the inner section 111, and also over outwardly-projecting ribs 113a on the inside face of the outer section 113, in order to assemble the three sections together. The upper wall of the intermediate section 112 is further formed with a resilient tongue 112b which, when the intermediate section is moved outwardly with respect to the inner section 111, rides over an annular rib 111b formed at the distal end of the inner section 111. The tip 112c of tongue 112b is bent inwardly such that when the intermediate section 112 has been moved to its outermost position with respect to the inner section 111, tongue 112b moves inwardly, under its inherent resiliency, to cause its tip 112c to engage the outer face of rib 111b, and thereby to prevent the intermediate section 112 from moving in the reverse direction (i.e., to its nested condition) with respect to the inner section 111.

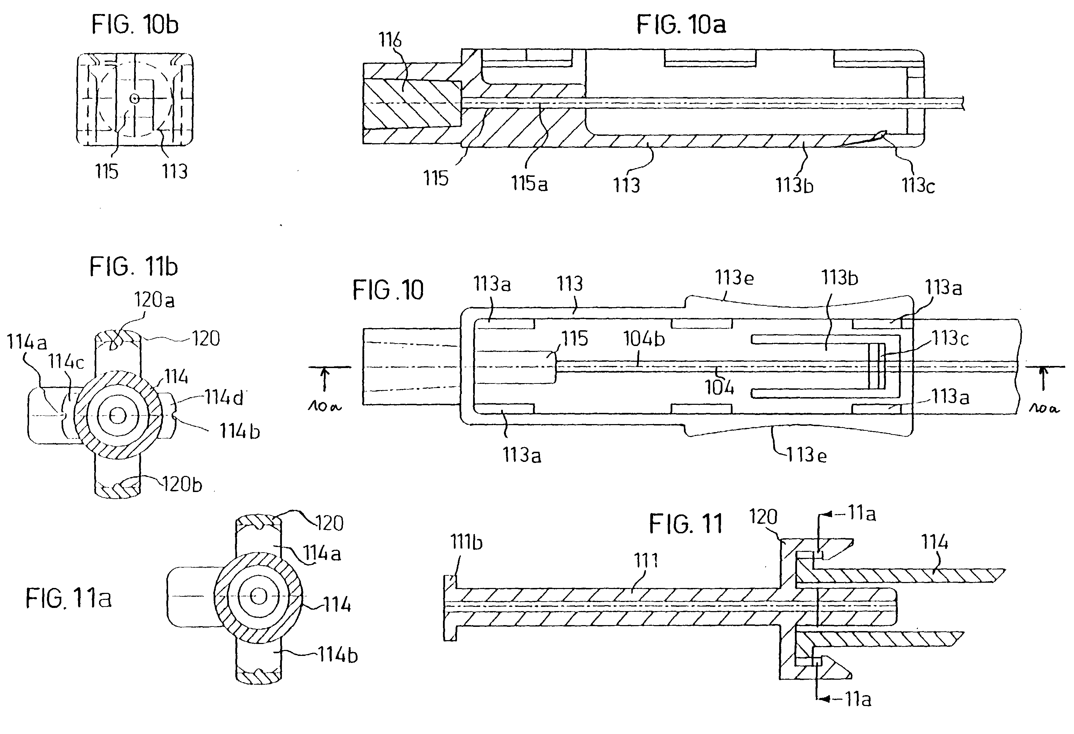

The outer section 113 (Fig. 10) of the protective enclosure 110 is also open at one side and is similarly formed with a resilient tongue 113b terminating in an inwardly-bent tip 113c. This tip also permits the outer section to move outwardly with respect to the intermediate section 112, and engages ribs 112d at the end of the intermediate section to prevent the outer section from moving in the opposite direction (towards its nested condition) with respect to the intermediate section 112.

The distal end 102b of the cannula 102 is also secured to one end of a cannula hub 114, as in Figs. 1-3. In this case, however, the opposite end of the cannula hub 114 is secured to the proximal end of the inner section 111 by means of a Leuer connection 120 (Fig. 11), which includes a pair of projections 120a, 120b receivable in recesses 114a, 114b formed in a pair of ears 114c, 114d fixed to the cannula hub 114. This Leuer connection permits the protective enclosure to be conveniently attached to or detached from the cannula hub by merely rotating one with respect to the other, as shown in Fig. 11a (attached condition) and Fig. 11b (detached condition).

The distal end 104b of the hollow needle 104 is secured to a needle hub 115 (Fig. 10) fixed to the outer telescoping section 113 of the protective enclosure 110, similar to the arrangement of Figs. 1-3. The outer face of the outer section 113 is dished at its opposite sides, as shown at 113e, to permit convenient gripping of that section between the user's thumb and second finger, while the user's index finger engages an upstanding post 111c (Figs. 7, 8) formed in the proximal end of the inner section 111. Bore 115a formed through the needle hub 115 is normally closed by hydrophobic plug 116, corresponding to plug 16 in Figs. 1-3.

The proximal end of the outer section 113 is further formed with a pair of stops 113d which are engageable with a pair of ribs 112d formed at the distal end of the intermediate section 112, to limit the outermost position of the outer section 113 with respect to the intermediate section 112. Similarly, the proximal end of the intermediate section 112 is formed with an inwardly-directed shoulder 112e, engageable with an outer rib 111b formed at the distal end of the inner section 111, to limit the outermost position of the intermediate section with respect to the inner section.

The cannula assembly illustrated in Figs. 4-11 is used in the following manner.

The assembly is normally supplied in the compacted condition illustrated in Fig. 4, wherein the guide needle 104 is in its operative position with its end 104a projecting through the end 102a of the cannula 102, and the protective enclosure 110 in its contracted, nested condition. The assembly would normally be packaged and sterilized in this condition.

When the assembly is to be used, the proximal end 104a of the needle 104, together with the proximal end 102a of the cannula 102, is inserted into the subject's vein. This is conveniently facilitated by gripping the dished surfaces 113e of the outer section 113 between the user's thumb and second finger, while the user's index finger engages the upstanding post 111c.

As soon as blood flow is observed, the outer telescoping section 113, together with its needle hub 115, is moved outwardly to extract the needle from the subject and also from the cannula 102. First, the outer section 113 moves outwardly with respect to the intermediate section 112, until tongue 113b snaps its tip 113c into engagement with the end of the intermediate section 112; and then the intermediate section 112 moves outwardly with respect to the inner section 111, until its tongue 112b snaps its tip 112c against the end of the inner section. Thus, when the user hears two clicks, indicating the snapping of the two tongues 113b and 112b, respectively, into engagement with the ends of the intermediate section 112 and inner section 111, respectively, the user is informed that the telescoping sections 111-113 of the protective enclosure 110 have moved to their extended protective condition, protecting the needle 104 which has been extracted from the cannula 102.
When the two tongues have thus snapped into place, they prevent the sections 111-113 from being moved in the opposite direction, towards their nested condition, which would dangerously expose the end of the needle 104.
The embodiments illustrated in Fig. 1-11 are herein included for information only and do not constitute the subject matter of the claims.

The cannula assembly illustrated in Figs. 12-23 is similar to those described above, in that it also includes an outer plastic cannula 202, an inner hollow guide needle 204, and a protective enclosure 210 for enclosing the guide needle when moved to its retracted position as illustrated in Figs. 14 and 15. The protective enclosure 210 also includes three telescopic sections 211, 212, 213, of transverse dimensions increasing from the inner section 211 to the outer section 213, as in the assembly of Figs. 4-11b. The outer section 213 is also formed with a needle hub 215 closed by a hydrophobic plug (not shown) corresponding to plug 16 in Figs. 1-3. In this case, however, the outer section 213 and intermediate section 212 are of rectangular configuration, as described above, but the inner section 211 is of cylindrical configuration to permit the inner section to rotate with respect to the remaining two sections 212, 213 at all times except when the enclosure is in its fully extended condition. As will be described more particularly below, this arrangement prevents detaching the hub assembly 214 from the Leuer connection 220 carried by the inner section 211 at all times except when the three sections of the enclosure are in their extended protective positions, to assure that the needle is protected against contact when the cannula assembly is removed.

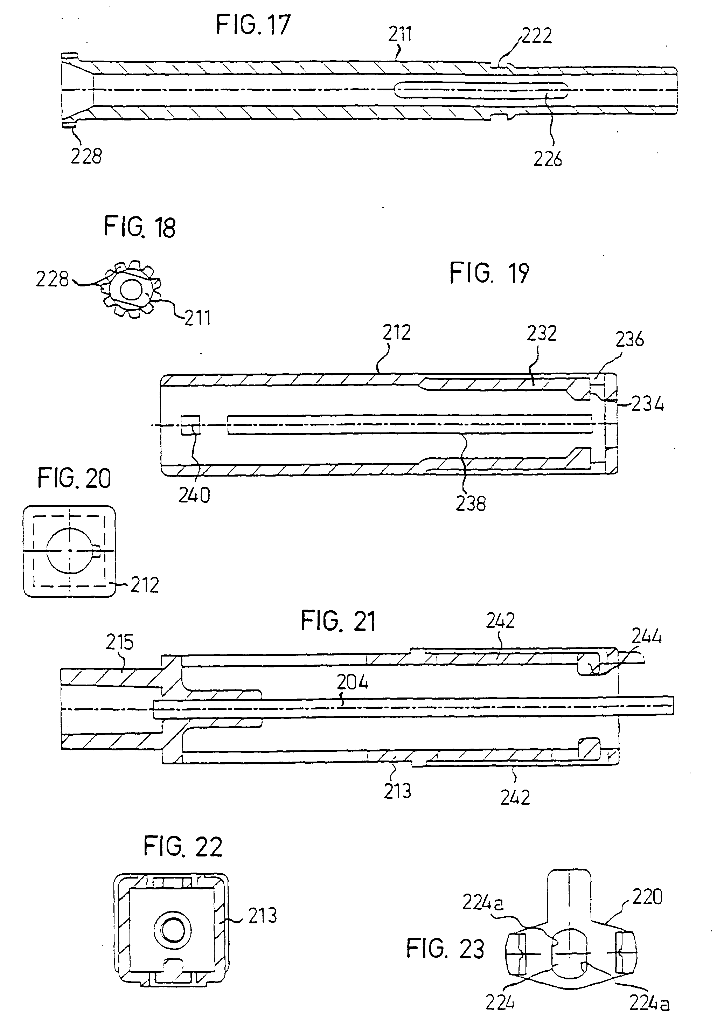

As shown particularly in Fig. 17, the inner section 211 is of hollow cylindrical configuration. At its proximal end, it is formed with an annular recess 222 for receiving the Leuer connector 220. For this purpose, the Leuer connector 220 (Fig. 23) is formed with a circular opening 224 substantially equal to, or slightly larger than, the outer diameter of recess 222, but flattened at its opposite ends 224a to decrease the size of that opening along that diameter. In addition, the proximal end of the inner section 211 is formed with a pair of axial slots 226 passing through the annular recess 222, to impart radial elasticity to this portion of the inner section 211 in the radial direction. Thus, the Leuer connector 220 may be inserted from the proximal end of the inner section 211 and snapped into the annular recess 222 so as to be firmly coupled to the inner section 211 and to rotate with it whenever the inner section rotates with respect to the remaining sections 212 and 213.

The distal end of the inner section 211 is formed with an annular array of teeth 228 which extend radially outwardly of the remainder of the section. These teeth cooperate with a pair of diametrically-opposed teeth 230 (Fig. 14) in the proximal end of the intermediate section 212.

The intermediate section 212 is further formed with a pair of resilient tongues 232 on its opposite sides, each having an inwardly-bent tip 234 terminating short of the respective end of the section to define a gap 236. Resilient tongues 232 permit the intermediate section 212 to be moved outwardly with respect to the inner section 211 until the annular teeth 228 of the inner section reach the proximal end of the intermediate section, at which time the end faces of the tongues 232 snap against the end faces of the annular teeth 228 to prevent axial movement of one section with respect to the other in the reverse direction. At this time, teeth 230 of the intermediate section 212 also seat between a pair of the annular teeth 228 in the inner section 211, and thereby also prevent rotary movement of one section with respect to the other.

The inner surface of the intermediate section 212 is further formed with a pair of axially-extending recesses 238, starting from the proximal end and terminating short of the distal end of the section. At its distal end, the intermediate section 212 is further formed with a pair of square holes 240 in alignment with the recesses 238.

The outer section 213 is similarly formed with a pair of resilient tongues 242, each terminating in an inwardly-bent tip 244. The tips 244 are received within recess 238 of the intermediate section 212 and guide the movement of the outer section with respect to the intermediate section until the two sections are in their fully extended condition, whereupon the tips 244 snap into the openings 240 in the intermediate section. When this occurs, the intermediate section 212 is locked to the outer section 213, with respect to both axial and rotary movements. Since the two telescoping sections 212 and 213 are of rectangular configuration, both sections are locked together during rotation so that they rotate together. However, since the inner section 211 is of cylindrical configuration, it can rotate relative to the intermediate section 212, except when the two sections are in their fully extended condition wherein the teeth 230 on the intermediate section 212 are received between the teeth 228 on the inner section 211.

The cannula assembly illustrated in Figs. 12-23 is otherwise constructed, and used, substantially in the same manner as described above with respect to the other two assemblies. However, the assembly of Figs. 12-23 provides better protection against accidental removal of the hub assembly 214 from the Leuer connector 220, except when the three-section enclosure 211, 212, 213 is in its fully extended position to completely enclose the needle 204.

Thus, the cannula hub 214 would normally be detached from the protective enclosure 210 by gripping the outer section 213 of the protective enclosure with one hand, gripping the cannula hub 214 with the other hand, and rotating one with respect to the other in order to detach the cannula hub from the Leuer connector 220 carried by the inner section 211. The tongues 242 of the outer section 213 are of softer resiliency than the tongues 232 of the intermediate section 212, so that when moving the outer section outwardly, that section will first move outwardly with respect to the intermediate section 212, and then the intermediate section will move outwardly with respect to the inner section 211. Until the intermediate section 212 has moved completely outwardly with respect to the inner section 211, rotation of the outer section 213 will not rotate the inner section 211 with respect to the Leuer connector 220, so that the cannula hub 214 will remain attached to the Leuer connector carried by the inner section. However, when the intermediate section 212 moves fully outwardly with respect to the inner section 211 (which, as described earlier, occurs after the outer section 213 has been moved fully to its outward position), the teeth 230 of the intermediate section seat between the teeth 228 of the inner section, thereby locking the two sections to rotate together, enabling the cannula hub 214 to be detached from the Leuer connector 220.

Once the three sections have been moved to their outermost, extended positions, they cannot thereafter be moved in the opposite direction. This is because the inwardly-bent tips 244 of the resilient tongues 242 in the outer section 213 are received within openings 240 in the intermediate section 212, and the inwardly-bent tips 234 of the resilient tongues 232 in the intermediate section 212 abut against the end of the inner section 211. The snapping of the tips of these two pairs of resilient tongues into their respective locking positions produces two "clicks", which provide a clear indication that the protective enclosure has been moved to its fully extended position for protecting the needle therein before the cannula hub is detached.

It will thus be seen that the use of any one of the cannula assemblies, as described above, does not hamper or substantially change the common practise of using such assemblies. Moreover, the protection of a contaminated needle is automatic and does not necessitate a special needle protection step. Each assembly also prevents re-extension of the needle from the protected configuration. Further, each assembly need not be substantially larger than current products; and finally, each assembly is simple to use and inexpensive to manufacture.

While the invention has been described with respect to two preferred embodiments, it will be appreciated that these are set forth merely for purposes of example, and that many other variations, modifications and applications of the invention may be made, within the protective scope defined by the appended claims.

## Claims

1. An intravenous cannula assembly, comprising:
a cannula (202) having a proximal end insertable through an opening in a subject's skin for draining fluid or for introducing a medication via its distal end;
a guide needle (204) movable to an operative position with respect to said cannula (202) and having a proximal end for piercing the subject's skin and for guiding the proximal end of the cannula through the subject's skin, said guide needle also being movable to a retracted position via the distal end of the cannula for removal therefrom;
and a protective enclosure (210) for enclosing the guide needle (204) when moved to its retracted position;
said protective enclosure (210) comprising a plurality of sections (211, 212, 213) having a total length to completely enclose said guide needle (204) when in its retracted position; said plurality of sections being movable from a compact nested condition in the operative position of the guide needle, to an extended protective condition in the retracted position of the guide needle; said sections including an outer section (213) to which the distal end of the needle is secured, and an inner section (211) detachably secured to a cannula hub (214) adjacent the distal end of said cannula;
wherein the distal end of said cannula is secured to one end of said cannula hub (214), the opposite end of said hub (214) having a rotatable connection with the inner section (211) of said protective enclosure, said inner section being rotatable with respect to said cannula hub (214) to attach said inner section to said cannula hub, and to detach said inner section from said cannula hub (214);
**characterized in that** it comprises rotatable coupling means which couples together the sections for rotation only in the extended condition of the enclosure, said rotatable coupling means including a tooth (230) at the proximal end of an intermediate section (212) co-operable with an annular array of teeth (228) at the distal end of said inner section; and
a pair of diametrically opposed ribs fixed to the inner section (211) of the protective enclosure (210) and a pair of diametrically opposed ribs fixed to the cannula hub (214), one of said pair of ribs being formed with radially extending projections receivable within recesses formed in said other pair of ribs, such that the protective enclosure (210), with the guide needle (204) enclosed therein, may be rotated in either direction to detach it from said cannula hub (214).

2. The assembly according to claim 1, wherein a tooth (244) is provided at the proximal end of said outer section receivable within an opening (240) in the distal end of the intermediate section.

3. The assembly according to any one of claims 1-2, wherein the proximal end of said inner section is formed with an annular recess (222) for receiving a Luer connector (220), and with a pair of axial slots (226) passing through said annular recess for imparting radial elasticity to said proximal end, and thereby to permit said Luer connector to be applied to the inner section from its proximal end.

4. The assembly according to claim 1, wherein said protective enclosure (210) includes blocking means carried by said plurality of sections (212, 213) of the protective enclosure permitting movement of said sections from said compact nested condition to said extended protective condition, but blocking movement of said sections from said extended protective condition to said compact nested condition.

5. The assembly according to claim 4, wherein said protective enclosure includes at least one intermediate section (212) between said inner and outer sections.

6. The assembly according to either of claims 4 or 5, wherein said blocking means comprises a resilient tongue (232, 242) on at least two of said sections having a free end engageable with an end of the adjacent section in the extended condition of the enclosure.

7. The assembly according to any one of claim 4-6, wherein the inner section of said protected enclosure has a passageway permitting the guide needle (204) to move in one direction therethrough, from its operative position to its retracted position; said assembly further including safety means preventing the guide needle from being moved in the opposite direction through said passageway, from its retracted position to its operative position.

## Patentansprüche

1. Eine intravenöse Kanüleneinheit, die folgendes umfaßt:
eine Kanüle (202), die ein proximales Ende hat, das durch eine Öffnung in die Haut eines Patienten gesteckt werden kann, um über ihr distales Ende ein Fluid abzuziehen oder ein Medikament einzuführen;
eine Führungsnadel (204), die in bezug auf die Kanüle (202) in eine Betriebsstellung bewegt werden kann und über ein proximales Ende verfügt, um in die Haut des Patienten zu stechen und um das proximale Ende der Kanüle durch die Haut des Patienten zu führen, wobei die Führungsnadel durch das distale Ende der Kanüle auch in eine eingezogene Stellung bewegt werden kann, um daraus entnommen zu werden;
und eine Schutzhülle (210) zum Umschließen der Führungsnadel (204), wenn sie in ihre eingezogene Stellung bewegt wird;
wobei die Schutzhülle (210) eine Mehrzahl an Abschnitten (211, 212, 213) umfaßt, die eine Gesamtlänge haben, um die Führungsnadel (204) vollständig zu umschließen, wenn sie sich in ihrer eingezogenen Stellung befindet; wobei die Mehrzahl an Abschnitten von einem kompakten ineinandergeschobenen Zustand in der Betriebsstellung der Führungsnadel in einen ausgebreiteten Schutzzustand in der eingezogenen Stellung der Führungsnadel bewegt werden kann; wobei die Abschnitte einen Außenabschnitt (213), an den das distale Ende der Nadel gesichert wird, und einen Innenabschnitt (211) einschließen, der lösbar an einer Kanülennabe (214) am distalen Ende der Kanüle angrenzend gesichert wird;
wobei das distale Ende der Kanüle an einem Ende der Kanülennabe (214) gesichert wird, wobei das entgegengesetzte Ende der Nabe (214) eine drehbare Verbindung mit dem Innenabschnitt (211) der Schutzhülle aufweist, wobei der Innenabschnitt in bezug auf die Kanülennabe (214) drehbar ist, um den Innenabschnitt an der Kanülennabe zu befestigen und um den Innenabschnitt von der Kanülennabe (214) zu lösen;
**dadurch gekennzeichnet, daß** sie ein drehbares Kopplungsmittel umfaßt, das die Abschnitte nur zum Drehen im ausgestreckten Zustand der Hülle aneinanderkoppelt, wobei das drehbare Kopplungsmittel einen Zahn (230) am proximalen Ende eines Zwischenabschnitts (212) einschließt, der mit einer ringförmigen Reihe von Zähnen (228) am distalen Ende des Innenabschnitts zusammenwirkt; und
dadurch daß, sie ein Paar von diametral entgegengesetzten Rippen umfaßt, die am Innenabschnitt (211) der Schutzhülle (210) befestigt sind, und ein Paar von diametral entgegengesetzten Rippen umfaß, die an der Kanülennabe (214) befestigt sind, wobei eines der Paare von Rippen mit sich radial erstreckenden Vorsprüngen ausgebildet ist, die innerhalb der Einschnitte aufgenommen werden können, die im anderen Paar von Rippen ausgebildet sind, so daß die Schutzhülle (210) mit der darin eingeschlossenen Führungsnadel (204) in beiden Richtungen gedreht werden kann, um sie von der Kanülennabe (214) zu lösen.

2. Die Einheit nach Anspruch 1, wobei ein Zahn (244) am proximalen Ende des Außenabschnitts bereitgestellt ist, der innerhalb einer Öffnung (240) im distalen Ende des Zwischenabschnitts aufgenommen werden kann.

3. Die Einheit nach irgendeinem der Ansprüche 1-2, wobei das proximale Ende des Innenabschnitts mit einem ringförmigen Einschnitt (222) zum Aufnehmen eines Luer-Anschlußglieds (220) und mit einem Paar von axialen Schlitzen (226) ausgebildet ist, die durch den ringförmigen Einschnitt passieren, um dem proximalen Ende eine radiale Elastizität zu verleihen und um dadurch dem Luer-Anschlußglied zu erlauben, am Innenabschnitt von seinem proximalen Ende aus angebracht zu werden.

4. Die Einheit nach Anspruch 1, wobei die Schutzhülle (210) ein von der Mehrzahl von Abschnitten (212, 213) der Schutzhülle getragenes Sperrmittel einschließt, um die Bewegung der Abschnitte aus dem kompakten ineinandergeschobenen Zustand in den ausgestreckten Schutzzustand zu erlauben, jedoch um die Bewegung der Abschnitte aus dem ausgestreckten Schutzzustand in den kompakten ineinandergeschobenen Zustand zu blockieren.

5. Die Einheit nach Anspruch 4, wobei die Schutzhülle mindestens einen Zwischenabschnitt (212) zwischen den Innen- und Außenabschnitten einschließt.

6. Die Einheit nach irgendeinem der Ansprüche 4 oder 5, wobei das Sperrmittel eine elastische Zunge (232, 242) an mindestens zwei der Abschnitte umfaßt, die ein freies Ende hat, das mit einem Ende des angrenzenden Abschnitts im ausgestreckten Zustand der Hülle in Eingriff bringbar ist.

7. Die Einheit nach irgendeinem der Ansprüche 4-6, wobei der Innenabschnitt der Schutzhülle einen Durchgang aufweist, der es der Führungsnadel (204) erlaubt, sich in einer Richtung dadurch von ihrer Betriebsstellung in ihre eingezogene Stellung zu bewegen; wobei die Einheit weiterhin ein Sicherheitsmittel einschließt, das es verhindert, daß die Führungsnadel von ihrer eingezogenen Stellung in ihre Betriebsstellung in der entgegengesetzten Richtung durch den Durchgang bewegt wird.

## Revendications

1. Système de canule intraveineuse, comprenant :
une canule (202) ayant une extrémité proximale insérable par une ouverture dans la peau d'un patient pour drainer un fluide ou pour introduire une médication via son extrémité distale ;
une aiguille de guidage (204) pouvant venir dans une position d'utilisation par rapport à ladite canule (202) et ayant une extrémité proximale pour percer la peau du patient et pour guider l'extrémité proximale de la canule à travers la peau du patient, ladite aiguille de guidage pouvant aussi venir en position de retrait par l'extrémité distale de la canule pour être retirée de celle-ci ;
et une enveloppe protectrice (210) destinée à contenir l'aiguille de guidage (204) lors de sa venue en position de retrait ;
ladite enveloppe protectrice (210) comportant une pluralité de portions (211, 212, 213) ayant une longueur totale permettant de contenir entièrement ladite aiguille de guidage (204) lorsqu'elle est dans sa position de retrait ; ladite pluralité de portions pouvant passer d'un état emboîté compact dans la position d'utilisation de l'aiguille de guidage à un état déployé protecteur dans la position de retrait de l'aiguille de guidage ; lesdites portions comprenant une portion extérieure (213), à laquelle est fixée l'extrémité distale de l'aiguille, et une portion intérieure (211) fixée de manière amovible à un pavillon (214) de canule adjacent à l'extrémité distale de ladite canule ;
l'extrémité distale de ladite canule étant fixée à une extrémité dudit pavillon (214) de canule, l'extrémité opposée dudit pavillon (214) ayant un raccordement tournant avec la portion intérieure (211) de ladite enveloppe protectrice, ladite portion intérieure pouvant tourner par rapport audit pavillon (214) de canule pour fixer ladite portion intérieure audit pavillon de canule, et pour détacher ladite portion intérieure par rapport audit pavillon (214) de canule ;
**caractérisé en ce qu'**il comporte un moyen d'accouplement rotatif qui accouple les portions pour qu'elles tournent uniquement dans l'état déployé de l'enveloppe, ledit moyen d'accouplement rotatif comportant, à l'extrémité proximale d'une portion intermédiaire (212), une dent (230) pouvant coopérer avec une série annulaire de dents (228) présente à l'extrémité distale de la dite portion intérieure ; et
une paire de nervures diamétralement opposées, fixées à la portion intérieure (211) de l'enveloppe protectrice (210) et une paire de nervures diamétralement opposées fixées au pavillon (214) de canule, une première nervure de ladite paire de nervures étant pourvue de saillies radiales pouvant être reçues dans des évidements ménagés dans ladite autre paire de nervures, de sorte qu'on peut faire tourner l'enveloppe protectrice (210) dans un sens ou dans l'autre, avec l'aiguille de guidage (204) à l'intérieur de celle-ci, pour la détacher dudit pavillon (214) de canule.

2. Système selon la revendication 1, dans lequel une dent (244) présente à l'extrémité proximale de ladite portion extérieure peut être reçue dans une ouverture (240) à l'extrémité distale de la portion intermédiaire.

3. Système selon l'une quelconque des revendications 1 et 2, dans lequel l'extrémité proximale de ladite portion intérieure comporte un évidement annulaire (222) destiné à recevoir un embout de raccordement Luer (220), et d'une paire de fente axiales (226) traversant ledit évidement annulaire pour donner une élasticité radiale à ladite extrémité proximale, et à permettre de ce fait le montage dudit embout Luer sur la portion intérieure depuis son extrémité proximale.

4. Système selon la revendication 1, dans lequel ladite enveloppe protectrice (210) comporte un moyen de blocage porté par une pluralité de portions (212, 213) de l'enveloppe protectrice permettant auxdites portions de passer dudit état emboîté compact audit état déployé protecteur, mais empêchant le passage desdites portions dudit état déployé protecteur audit état emboîté compact.

5. Système selon la revendication 4, dans lequel ladite enveloppe protectrice comporte au moins une portion intermédiaire (212) entre lesdites portions intérieure et extérieure.

6. Système selon l'une ou l'autre des revendications 4 et 5, dans lequel ledit moyen de blocage comportant sur au moins deux desdites portions une languette élastique (232, 242) ayant une extrémité apte à coopérer avec une extrémité de la portion adjacente dans l'état déployé de l'enveloppe.

7. Système selon l'une quelconque des revendications 4 à 6, dans lequel la portion intérieure de ladite enveloppe protectrice comporte un passage permettant à l'aiguille de guidage (204) de passer dans un sens dans celui-ci, de sa position d'utilisation à sa position de retrait, ledit système comportant en outre un moyen de sécurité empêchant l'aiguille de guidage de passer en sens inverse, dans ledit passage, de sa position de retrait à sa position d'utilisation.
